# NOUVEAU FASCICULE DE BREVET EUROPEEN

(11) **EP 0 698 108 B2**
(45) Date de publication et mention de la décision concernant l'opposition: **21.12.2005**
(45) Mention de la délivrance du brevet: 28.11.2001
(21) Numéro de dépôt: 94916259.8
(22) Date de dépôt: 06.05.1994
(51) Int. Cl.: C12N 15/86, C12N 15/26, C07K 14/075, A61K 48/00, C12N 7/01, A61K 39/235

(54) **VECTEURS ADENOVIRAUX D'ORIGINE ANIMALE ET UTILISATION EN THERAPIE GENIQUE**
ADENOVIRALE VEKTOREN TIERISCHEN URSPRUNGS UND IHRE VERWENDUNG BEI DER GENTHERAPIE
ADENOVIRAL VECTORS OF ANIMAL ORIGIN AND USE THEREOF IN GENE THERAPY

(30) Priorité: 18.05.1993 FR 9305954
(43) Date de publication de la demande: 28.02.1996
(73) Titulaire: CENTELION, 94400 Vitry Sur Seine (FR)
(72) Inventeur: HADDADA, Hedi, F-94140 Alfortville (FR); KLONJKOWSKI, Bernard, F-93140 Bondy (FR); PERRICAUDET, Michel, F-28320 Ecrosnes (FR); VIGNE, Emmanuelle, F-94200 Ivry (FR)
(74) Mandataire: Lecca, Patricia S.
(86) Numéro de dépôt international: PCT/FR1994/000531
(87) Numéro de publication internationale: WO 1994/026914

(56) Documents cités:
- WO-A-91/11525
- WO-A-93/06223
- WO-A-93/19191

## Description

La présente invention concerne de nouveaux vecteurs viraux, leur préparation et leur utilisation en thérapie génique. Elle concerne également les compositions pharmaceutiques contenant lesdits vecteurs viraux. Plus particulièrement, la présente invention concerne l'utilisation d'adénovirus recombinants d'origine animale comme vecteurs pour la thérapie génique.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Parmi ces virus, les adénovirus présentent certaines propriétés intéressantes pour une utilisation en thérapie génique. Notamment, ils ont un spectre d'hôte assez large, sont capables d'infecter des cellules quiescentes, et ils ne s'intègrent pas au génome de la cellule infectée. Pour ces raisons, les adénovirus ont déjà été utilisés pour le transfert de gènes in vivo. A cet effet, différents vecteurs dérivés des adénovirus ont été préparés, incorporant différents gènes (β-gal, OTC, α-1AT, cytokines, etc). Tous les vecteurs dérivés des adénovirus décrits dans l'art antérieur en vue d'une utilisation en thérapie génique chez l'homme ont jusqu'à présent été préparés à partir d'adénovirus d'origine humaine. Ceux-ci semblaient en effet les plus appropriés pour une telle utilisation. Pour limiter les risques de multiplication et la formation de particules infectieuses in vivo, les adénovirus utilisés sont généralement modifiés de manière à les rendre incapables de réplication dans la cellule infectée. Ainsi, les constructions décrites dans l'art antérieur sont des adénovirus délétés des régions E1 (E1a et/ou E1b) et éventuellement E3 au niveau desquelles sont insérées des séquences d'intérêt (Levrero et al., Gene 101 (1991) 195; Gosh-Choudhury et al., Gene 50 (1986) 161). Cependant, en plus de cette étape nécessaire de modification, les vecteurs décrits dans l'art antérieur conservent d'autres inconvénients qui limitent leur exploitation en thérapie génique, et notamment des risques de recombinaison avec des adénovirus sauvages. La présente invention apporte une solution avantageuse à ce problème.

La présente invention réside en effet dans l'utilisation d'adénovirus recombinants d'origine animale non-humaine dont le génome est dépourvu des séquences nécessaires à la réplication, et contenant une séquence d'ADN hétérologue pour la préparation d'une composition pharmaceutique destinée au traitement thérapeutique et/ou chirurgical du corps humain. La présente invention résulte de la mise en évidence que les adénovirus d'origine animale sont capables d'infecter avec une grande efficacité les cellules humaines. L'invention repose également sur la mise en évidence que les adénovirus d'origine animale sont incapables de se propager dans les cellules humaines dans lesquelles ils ont été testés. L'invention repose enfin sur la découverte surprenante que les adénovirus d'origine animale ne sont nullement trans-complémentés par des adénovirus d'origine humaine, ce qui élimine tout risque de recombinaison et de propagation in vivo, en présence d'un adénovirus humain, pouvant conduire à la formation d'une particule infectieuse. Les vecteurs de l'invention sont donc particulièrement avantageux puisque les risques inhérents à l'utilisation de virus comme vecteurs en thérapie génique tels que la pathogénicité, la transmission, la réplication, la recombinaison, etc sont fortement réduits voire supprimés.

La présente invention fournit ainsi des vecteurs viraux particulièrement adaptés au transfert et/ou à l'expression chez l'homme de séquences d'ADN désirées.

Un premier objet de la présente invention concerne donc l'utilisation d'un adénovirus recombinant d'origine animale non-humaine dont le génome est dépourvu des séquences nécessaires à la réplication, et contenant une séquence d'ADN hétérologue pour la préparation d'une composition pharmaceutique destinée au traitement thérapeutique et/ou chirurgical du corps humain.

Les adénovirus d'origine animale utilisables dans le cadre de la présente invention peuvent être d'origine diverses, à l'exclusion des adénovirus humains. Les adénovirus humains sont ceux naturellement infectieux chez l'homme, généralement désignés par le terme HAd ou Ad.

En particulier, les adénovirus d'origine animale utilisables dans le cadre de la présente invention peuvent être d'origine canine, bovine, murine, (exemple : Mavl, Beard et al., Virology 75 (1990) 81), ovine, porcine, aviaire ou encore simienne (exemple : SAV).

Plus particulièrement, parmi les adénovirus aviaires, on peut citer les sérotypes 1 à 10 accessibles à l'ATCC, comme par exemple les souches Phelps (ATCC VR-432), Fontes (ATCC VR-280), P7-A (ATCC VR-827), IBH-2A (ATCC VR-828), J2-A (ATCC VR-829), T8-A (ATCC VR-830), K-11 (ATCC VR-921) ou encore les souches référencées ATCC VR-831 à 835. Parmi les adénovirus bovins, on peut utiliser les différents sérotypes connus, et notamment ceux disponibles à l'ATCC (types 1 à 8) sous les référencesATCC VR-313, 314, 639-642, 768 et 769. On peut également citer les adénovirus murins FL (ATCC VR-550) et E20308 (ATCC VR-528), l'adénovirus ovin type 5 (ATCC VR-1343), ou type 6 (ATCC VR-1340); l'adénovirus porcin 5359), ou les adénovirus simiens tels que notamment les adénovirus référencée à l'ATCC sous les numéros VR-591-594, 941-943, 195-203, etc.

De préférence, on utilise dans le cadre de l'invention des adénovirus d'origine canine, et notamment toutes les souches des adénovirus CAV2 [souche manhattan ou A26/61 (ATCC VR-800) par exemple]. Les adénovirus canins ont fait l'objet de nombreuses études structurales. Ainsi, des cartes de restriction complètes des adénovirus CAV1 et CAV2 ont été décrites dans l'art antérieur (Spibey et al., J. Gen. Virol. 70 (1989) 165), et les gènes E1a, E3 ainsi que les séquences ITR ont été clonés et séquencés (voir notamment Spibey et al., Virus Res. 14 (1989) 241; Linné, Virus Res. 23 (1992) 119, WO 91/11525). Par ailleurs, les adénovirus canins ont déjà été utilisés pour la préparation de vaccins destinés à immuniser les chiens contre la rage, les parvovirus, etc (WO 91/11525). Cependant, jusqu'à présent, la possibilité d'utiliser ces adénovirus pour une thérapie génique chez l'homme n'a jamais été suggérée dans l'art antérieur. De plus, les intérêts d'une telle utilisation n'avaient jamais été mesurés.

Les adénovirus utilisés dans le cadre de l'invention doivent de préférence être défectifs, c'est-à-dire incapables de se propager de façon autonome dans l'organisme dans lequel ils sont administrés. Comme indiqué plus haut, la demanderesse a montré que les adénovirus d'origine animale sont capables d'infecter les cellules humaines, mais pas de s'y propager. En ce sens, ils sont donc naturellement défectifs chez l'homme et, contrairement à l'utilisation d'adénovirus humains, ne nécessitent pas de modification génétique à cet égard. Toutefois, le caractère défectif de ces adénovirus peut être amplifié par des modifications génétiques du génome, et notamment par modification des séquences nécessaires à la réplication dudit virus dans les cellules. Ces régions peuvent être soit éliminées (en tout ou en partie), soit rendues non-fonctionnelles, soit modifiées par insertion d'autres séquences et notamment de la séquence d'ADN hétérologue.

Selon l'origine de l'adénovirus, les séquences nécessaires à la réplication peuvent varier quelque peu. Néanmoins, elles sont généralement localisées près des extrêmités du génome. Ainsi, dans le cas de CAV-2, la région E1a a été identifiée, clonée et séquencée (Spibey et al., Virus Res. 14 (1989) 241). Celle-ci est située sur le fragment de 2 kb à l'extremité gauche du génome de l'adénovirus.

Par ailleurs, d'autres modifications génétiques peuvent être effectuées sur ces adénovirus, notamment pour éviter la production de protéines virales indésirables chez l'homme, pour permettre l'insertion de séquences d'ADN hétérologue de taille importante, et/ou pour insérer des régions déterminées du génome d'un autre adénovirus animal ou humain (exemple : gène E3).

Au sens de la présente invention, le terme "séquence d'ADN hétérologue" désigne toute séquence d'ADN introduite dans le virus, dont le transfert et/ou l'expression chez l'homme est recherché.

En particulier, la séquence d'ADN hétérologue peut comporter un ou plusieurs gènes thérapeutiques et/ou un ou plusieurs gènes codant pour des peptides antigéniques.

Dans un mode particulier de mise en oeuvre, l'invention concerne donc plus particulièrement l'utilisation d'adénovirus d'origine animale pour la préparation de compositions pharmaceutiques destinées au transfert de gènes thérapeutiques chez l'homme. Les gènes thérapeutiques qui peuvent ainsi être transférés sont tout gène dont la transcription et éventuellement la traduction dans la cellule cible génèrent des produits ayant un effet thérapeutique.

Il peut s'agir en particulier de gènes codant pour des produits protéiques ayant un effet thérapeutique. Le produit protéique ainsi codé peut être une protéine, un peptide, un acide aminé, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule lui permettant de lutter contre une pathologie.

Parmi les produits thérapeutiques au sens de la présente invention, on peut citer plus particulièrement les enzymes, les dérivés sanguins, les hormones, les lymphokines : interleukines, interférons, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc.

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Comme indiqué plus haut, la séquence d'ADN hétérologue peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'epstein barr, du virus de l'hépatite B (EP 185 573), du virus de la pseudo-rage, ou encore spécifiques de tumeurs (EP 259 212).

Généralement, la séquence d'ADN hétérologue comprend également des séquences permettant l'expression du gène thérapeutique et/ou du gène codant pour le peptide antigénique dans la cellule infectée. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Par ailleurs, la séquence d'ADN hétérologue peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Les adénovirus défectifs selon l'invention peuvent être préparés par toute technique connue de l'homme du métier. En particulier, ils peuvent être préparés selon le protocole décrit dans la demande WO 91/11525. La technique classique de préparation repose sur la recombinaison homologue entre un adénovirus animal et un plasmide portant entre autre la séquence d'ADN hétérologue que l'on désire insérer. La recombinaison homologue se produit après co-transfection desdits adénovirus et plasmide dans une lignée cellulaire appropriée. La lignée cellulaire utilisée doit de préférence être transformable par lesdits éléments, et, dans la cas où l'on utilise un adénovirus d'origine animale modifié, la lignée cellulaire peut si nécessaire comporter des séquences capables de complémenter la partie du génome de l'adénovirus défectif, de préférence sous forme intégrée pour éviter les risques de recombinaison. A titre d'exemple de lignée, on peut mentionner la lignée de cellules rénales de lévrier GHK (Flow laboratories) ou la la lignée cellulaire MDCK. Les conditions de culture des cellules et de préparation des virus ou de l'ADN viral ont également été décrites dans la littérature (voir notamment Macatney et al., Science 44 (1988) 9; Fowlkes et al., J. Mol. Biol. 132 (1979) 163).

Ensuite, les vecteurs qui se sont multipliés sont récupérés et purifiés selon les techniques classiques de biologie moléculaire.

Un autre objet de la présente invention réside dans un adénovirus recombinant d'origine animale non-humaine caractérisé en ce que son génome est dépourvu au moins des séquences nécessaires à la réplication et contenant au moins un gène inséré codant pour un produit protéique ayant un effet thérapeutique chez l'homme, choisi parmi les enzymes, les dérivés sanguins, les hormones, les lymphokines, les facteurs de croissance, les neurotransmetteurs ou leurs précurseurs ou enzymes de synthèse, les facteurs trophiques, les apolipoprotéines, la dystrophine ou une minidystrophine, les suppresseurs de tumeurs et les facteurs impliqués dans la coagulation. L'invention concerne également un adénovirus recombinant d'origine animale non-humaine contenant au moins une séquence antisens insérée ayant un effet thérapeutique chez l'homme.

L'invention a également pour objet toute composition pharmaceutique contenant un adénovirus recombinant d'origine animale tel que défini ci-dessus. Les compositions pharmaceutiques de l'invention peuvent être formulées en vue d'une administration par voie topique, orale, parentérale, intranasale, intraveineuse, intramusculaire, sous-cutanée, intraoculaire, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 104 et 1014 pfu/ml, et de préférence 106 à 1010 pfu/ml. Le terme pfu ("plaque forming unit") correspond au pouvoir infectieux d'une solution de virus, et est déterminé par infection d'une culture cellulaire appropriée, et mesure, généralement après 5 jours, du nombre de plages de cellules infectées. Les techniques de détermination du titre pfu d'une solution virale sont bien documentées dans la littérature.

Selon la séquence d'ADN hétérologue insérée, les adénovirus de l'invention peuvent être utilisés pour le traitement ou la prévention de nombreuses pathologies, incluant les maladies génétiques (dystrophie, fibrose cystique, etc), les maladies neurogégénératives (alzheimer, parkinson, ALS, etc), les cancers, les pathologies liées aux désordres de la coagulation ou aux dyslipoprotéinémies, les pathologies liées aux infections virales (hépatites, SIDA, etc), etc.

La présente invention sera plus complètement décrite à l'aide des exemples qui suivent, qui doivent être considérés comme illustratifs et non limitatifs.

### Légende des figures

Figure 1 : Carte de restriction de l'adénovirus CAV2 souche Manhattan (d'après Spibey et al précité).
Figure 2 : Carte des plasmides p1, p2 (figure 2a) et p3 (figure 2b).
Figure 3 : Stratégie de construction d'un adénovirus canin recombinant contenant une séquence d'ADN hétérologue.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

### E1. Infection de cellules humaines par des adénovirus d'origine canine.

Cet exemple démontre la capacité des adénovirus d'origine animale (canine) d'infecter des cellules humaines.

### E1.1. Lignées cellulaires utilisées

Dans cet exemple, les lignées cellulaires suivantes ont été utilisées :
- Lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %).
- Lignée de cellules humaines KB : Issue d'un carcinome épidermique humain, cette lignée est accessible à l'ATCC (ref. CCL17) ainsi que les conditions permettant sa culture.
- Lignée de cellules humaines Hela : Issue d'un carcinome de l'épithelium humain, cette lignée est accessible à l'ATCC (ref CCL2) ainsi que les conditions permettant sa culture.
- Lignée de cellules canines MDCK : Les conditions de culture des cellules MDCK ont été décrites notamment par Macatney et al., Science 44 (1988) 9.

### E1.2. Infection

Les cellules des lignées celulaires mentionnées ci-dessus ont été infectées par le virus CAV2 (souche Manhattan). Pour cela, les cellules (environ 10⁷ / boite) ont été incubées pendant 1 heure à 37°C en présence de 10 pfu / cellule de virus. Ensuite, 5 ml de milieu de culture ont été ajoutés et la culture est poursuivie à 37°C pendant 48 heures environ. A cet instant, l'ADN présent sous forme épisomique dans les cellules infectées a été analysé : Les résultats obtenus montrent que toutes ces lignées cellulaires présentent de l'ADN de CAV2 dans leurs noyaux, ce qui démontre leur infectabilité par les adénovirus canins.

### E2. Absence de propagation des adénovirus d'origine canine dans les cellules humaines.

Cet exemple démontre que les adénovirus canins, bien que capables d'infecter les cellules humaines, ne se propagent pas dans celles-ci.

Après infection des cellules selon l'exemple 1, la quantité d'ADN de CAV2 a été dosée au cours du temps selon le protocole suivant : L'ADN épisomique présent dans les cellules a été récupéré selon la technique décrite par Hirt et al. (J. Virol. 45 (1983) 91), et la quantité d'ADN a été dosée par comparaison avec une gamme étalon. Les résultats obtenus montrent que la quantité d'ADN viral n'augmente pas dans les cellules KB et 293, démontrant une absence totale de réplication de CAV2 dans ces cellules. Dans les cellules MDCK et Hela, une légère augmentation de la quantité d'ADN viral de CAV2 est observée. Cependant, le dosage de la formation de particules virales montre qu'aucune propagation de CAV2 ne se produit dans les cellules humaines 293, KB et Hela, mais uniquement dans la lignée canine MDCK. La propagation a été mesurée par récolte des cellules infectées, libération des éventuels virus par congélation/décongélation, et infection des cellules MDCK avec le surnageant ainsi obtenu, dans les conditions décrites précédemment. Après 48 heures de culture, l'absence d'ADN viral dans les cellules MDCK ainsi infectées démontre qu'aucune propagation virale a eu lieu dans les cellules humaines.

Ces résultats montrent clairement que les adénovirus canins sont incapables de se propager dans les cellules humaines.

### E3. Mise en évidence de l'absence de trans-complémentation des adénovirus canins par les adénovirus humains.

Cet exemple montre que l'absence de propagation des adénovirus canins dans les cellules humaines n'est pas trans-complémentée par la présence d'adénovirus humains.

Les cellules des lignées humaines 293, KB et Hela, et de la lignée canine MDCK ont été co-infectées par l'adénovirus CAV2 et l'adénovirus humain Ad5. La présence d'ADN viral (canin et humain) dans les cellules a été mise en évidence comme dans l'exemple E1, et la quantité d'ADN a été dosée au cours du temps ainsi que la propagation. Les résultats obtenus indiquent que la quantité d'ADN de CAV2 n'augmente pas dans le temps dans les cellules KB et 293, ce qui démontre que la présence de l'adénovirus humain Ad5 n'induit pas, par trans-complémentation, la réplication de CAV2 dans ces cellules. L'absence d'ADN viral dans les cellules MDCK infectées par les éventuels virus issus des cellules KB, 293 et Hela démontre de la même façon qu'aucune propagation de l'adénovirus CAV2 dans les lignées cellulaires humaines ne s'est produite, même en présence d'adénovirus humain. **E4. Construction d'une banque d'ADN génomique de CAV2.**

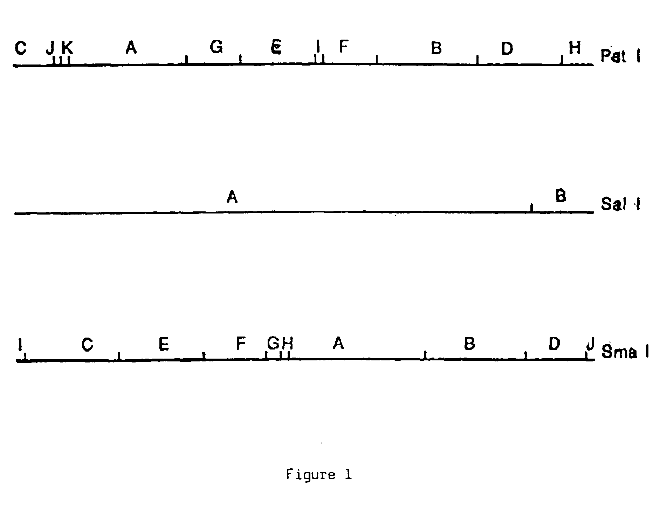

Une banque de plasmides a été constituée à partir de fragments de restriction du génome de l'adénovirus CAV2. Cette banque a été obtenue par digestion de CAV2 par les enzymes Smal et Pstl et clonage des fragments Smal : A, B, C, D, E, F, I, et J et Pstl : A, B, C, D, E, F, G, H (figure 1) dans le vecteur pGem3Zf+ (Promega). Le plasmide portant le fragment Smal C a ensuite été cotransfecté dans les cellules MDCK avec le plasmide pUC4KIXX (Pharmacia) portant le gène de résistance à la néomycine, pour établir une lignée MDCK exprimant constitutivement les gènes E1A et E1B de CAV2. Cette lignée permet la construction de virus recombinants délétés pour ces régions (CfE5.2.).

### E5. Construction d'adénovirus recombinants canins portant le gène de l'interleukine-2 sous contrôle du promoteur MLP d'Ad2.

Deux stratégies de construction d'adénovirus recombinants canins portant le gène de l'interleukine-2 sous contrôle du promoteur MLP d'Ad2 humain ont été élaborées.

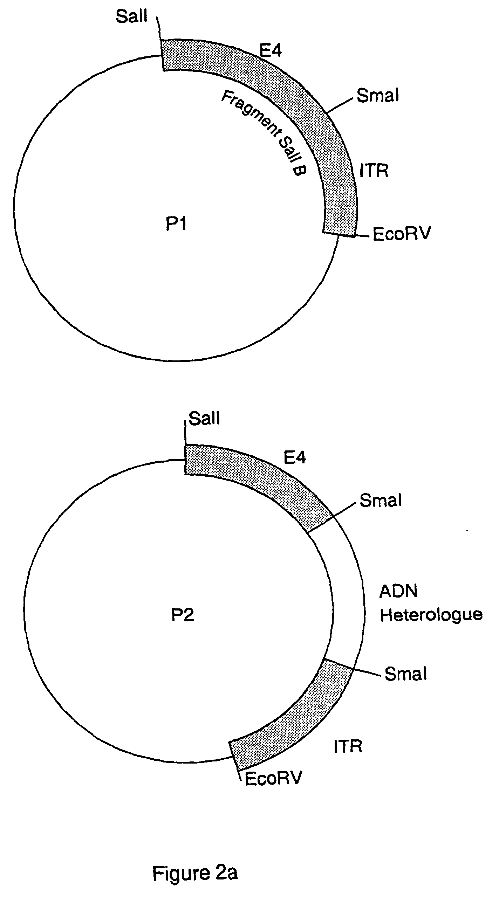

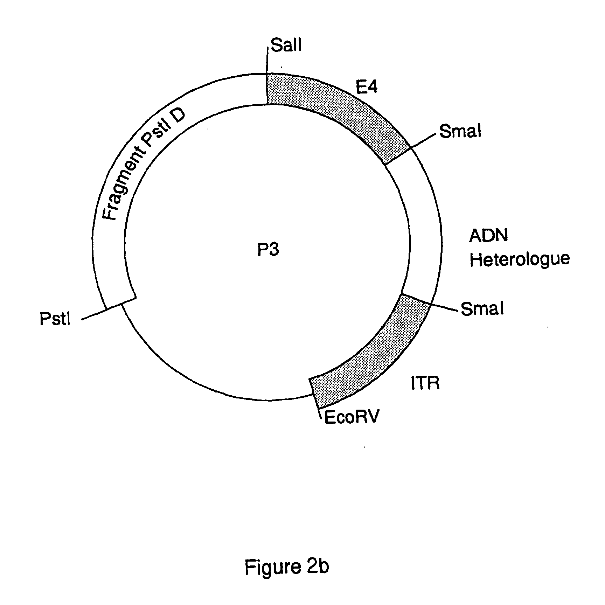

E5.1. La première consiste en l'insertion de la séquence d'ADN hétérologue désirée (promoteur MLP - gène de l'interleukine-2) entre la région E4 et l'ITR droite du génome entier de CAV2, au niveau du site de restriction Smal. La construction d'un tel recombinant est effectuée soit par ligation, soit par recombinaison in vivo entre un plasmide portant la séquence d'ADN hétérologue désirée et le génome de CAV2. Les plasmides utilisés pour l'obtention des adénovirus recombinants portant le gène de l'interleukine-2 sous contrôle du promoteur MLP sont construits de la manière suivante (figure 2):
- un premier plasmide, désigné p1 est obtenu par clonage du fragment Sall B de CAV2 (figue 1) contenant notamment l'ITR droite, un site Smal et le gène E4, dans le plasmide pGem3Zf+ (Promega), le site Smal est unique sur p1;
- la séquence d'ADN hétérologue (promoteur MLP - gène de l'interleukine-2) est introduite au niveau du site Smal du plasmide p1 pour générer le plasmide p2; et
- le fragment PstI-SalI contenu dans le fragment Pstl D de la banque génomique et portant une partie du gène E3 est ensuite cloné au site correspondant dans p2 pour générer le plasmide p3 (figure 2).

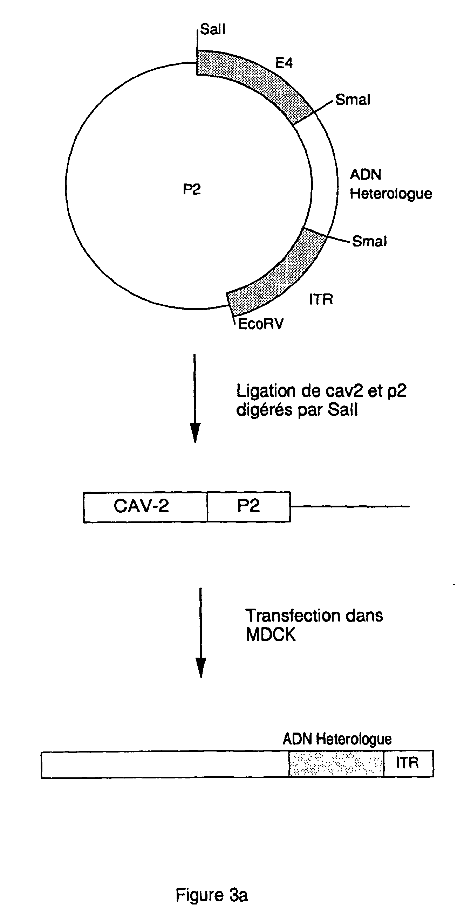

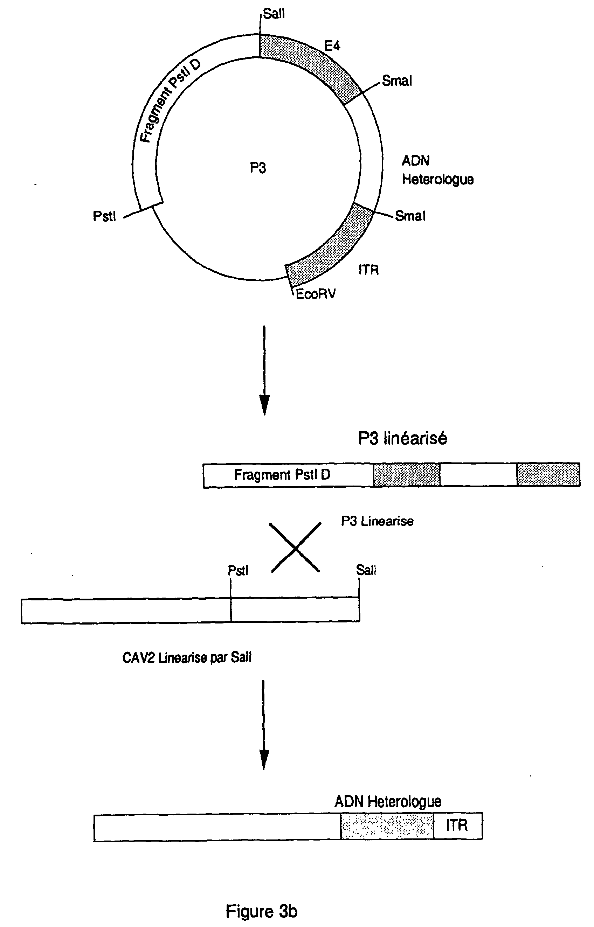

Les plasmides ainsi obtenus sont utilisés pour préparer les adénovirus recombinants, selon les deux protocoles suivants (voir figure 3) :
a) Ligation in vitro du plasmide p2 digéré par Sall au fragment Sall A de CAV2, et transfection du produit de ligation dans les cellules MDCK (figure 3a),
b) Recombinaison entre le plasmide p3 et le fragment Sall A de CAV2, après cotransfection dans les cellules MDCK (figure 3b). Les adénovirus recombinants obtenus sont ensuite isolés et amplifiés selon les techniques connues de l'homme du métier.

Ces manipulations permettent la construction d'adénovirus recombinants canins portant le gène de l'interleukine-2, utilisables pour le transfert de ce gène thérapeutique chez l'homme (figure 3).

E5.2. La seconde stratégie élaborée repose sur l'utilisation de la lignée cellulaire MDCK exprimant constitutivement les gènes E1A et E1B de CAV2 (Cf exemple E4). Cette lignée permet en effet detrans-complémenter des adénovirus canins délétés de ces régions, et ainsi, la construction de virus recombinants dans lesquels la séquence d'ADN hétérologue est substituée à la région E1. Pour cela, un plasmide comportant l'extrémité gauche (séquence ITR et séquence d'encapsidation) du génome de CAV2 et la séquence d'ADN hétérologue est construit. Ce plasmide est cotransfecté dans les cellules MDCK décrites ci-dessus, en présence du génome d'un adénovirus CAV2, délété de sa propre extrémité gauche. Les adénovirus canins recombinants produits sont récupérés, éventuellement amplifiés, et conservés en vue de leur utilisation chez l'homme.

## Revendications

1. Utilisation d'un adénovirus recombinant d'origine animale non-humainc dont le génome est dépourvu des séquences nécessaires à la réplication, et contenant une séquences d'ADN hétérologue pour la préparation d'une composition pharmaceutique destinée au traitement thérapeutique et/ou chirurgical du corps humain.

2. Utilisation selon la revendication 1 pour la préparation d'une composition pharmaceutique destinée au transfert de gènes thérapeutiques chez l'homme.

3. Utilisation selon la revendication 1 pour la préparation d'un vaccin.

4. Utilisation selon la revendication 2 **caractérisés en ce que** le gène thérapeutique est un gène codant pour un produit protéique thérapeutique.

5. Utilisation selon la revendication 2 **caractérisée en ce que** le gène thérapeutique est un gène ou une séquence antisens.

6. Utilisation selon l'une des revendications 1 à 5 **caractérisée en ce que** l'adénovirus est choisi parmi les adénovirus canin, bovin, murin, ovin, porcin, aviaire et simien.

7. Utilisation selon la revendication 6 **caractérisée en ce que** l'adénovirus est un adénovirus canin, de préférence choisi parmi les souches de l'adénovirus CAV-2.

8. Utilisation selon la revendication 7 **caractérisée en ce que** le génome de l'adénovirus est dépourvu des régions E1A et E1B.

9. Adénovirus recombinant d'origine animale non-humaine **caractérisé en ce que** son génome est dépourvu au moins des séquences nécessaires à la réplication et contenant au moins un gène inséré codant pour un produit protéique ayant un effet thérapeutique chez l'homme, choisi parmi les enzymes, les dérivés sanguins, les hormones, les lymphokines, les facteurs de croissance, les neurotransmetteurs ou leurs précurseurs ou enzymes de synthèse, les facteurs trophiques, les apolipoprotéines, la dystrophine ou une minidystrophine, les suppresseurs de tumeurs et les facteurs impliqués dans la coagulation.

10. Adénovirus recombinant d'origine animale non-humaine contenant au moins une séquence antisens insérée ayant un effet thérapeutique chez l'homme.

11. Adénovirus selon les revendications 9 ou 10 **caractérisée en ce qu'**il comprend également des séquences promotrices permettant l'expression du ou des gènes thérapeutiques insérés.

12. Adénovirus selon les revendications 9 à 11 **caractérisé en ce qu'**il comprend également des séquences signal permettent d'induire une sécrétion du produit d'expression du gène thérapeutique chez l'homme.

13. Adénovirus selon les revendications 9 à 12 **caractérisé en ce que** l'adénovirus est choisi parmi les adénovirus canin, bovin, murin, ovin, porcin, aviaire et simien.

14. Adénovirus selon la revendication 13 **caractérisée en ce que** l'adénovirus est un adénovirus canin, de préférence choisi parmi les souches de l'adénovirus CAV-2.

15. Adénovirus selon la revendication 14 **caractérisé en ce qu'**il comprend des régions d'un autre adénovirus animal ou humain.

16. Composition pharmaceutique comprenant un ou plusieurs adénovirus selon l'une des revendications 9 à 15.

## Patentansprüche

1. Verwendung eines rekombinanten Adenovirus mit Ursprung aus einem nicht-menschlichen Tier, dass dem Genom des Adenovirus die zur Replikation notwendigen Sequenzen fehlen, das eine hetcrologe DNA-Sequenz enthält, zur Herstellung einer pharmazeutischen Zusammensetzung zur therapeutischen und/oder chirurgischen Behandlung des menschlichen Körpers.

2. Verwendung nach Anspruch 1 zur Herstellung einer therapeutischen Zusammensetzung zum Transfer von therapeutischen Genen beim Menschen.

3. Verwendung nach Anspruch 1 zur Herstellung eines Impfstoffs.

4. Verwendung nach Anspruch 2, **dadurch gekennzeichnet, dass** das therapeutische Gen ein Gen ist, das ein therapeutisches Proteinprodukt codiert.

5. Verwendung nach Anspruch 2, **dadurch gekennzeichnet, dass** das therapeutische Gen ein Gen oder eine Antisensesequenz ist.

6. Verwendung nach einem der Ansprüche 1 bis 5, **dadurch gekennzeichnet, dass** das Adonovirus aus Hunde-, Rinder-, Maus-, Schaf-, Schweine-, Vogel- und Affen-Adenoviren ausgewählt ist.

7. Verwendung nach Anspruch 6, **dadurch gekennzeichnet, dass** das Adenovirus ein Hunde-Adenovirus, bevorzugt ausgewählt aus den Stämmen des Adenovirus CAV-2, ist.

8. Verwendung nach Anspruch 7, **dadurch gekennzeichnet, dass** dem Genom des Adenovirus die Regionen E1A und E1B fehlen.

9. Rekombinantes Adenovirus mit Ursprung aus einem nicht-menschlichen Tier, **dadurch gekennzeichnet, dass** dem Genom des Adenovirus mindenstens die zur Replikation notwendigen Sequenzen fehlen, enthaltend mindestens ein insertiertes Gen, das ein Proteinprodukt mit therapeutischer Wirkung beim Menschen, ausgewählt aus Enzymen, Blutderivaten, Hormonen, Lymphokinen, Wachstumsfaktoren, Neurotransmittern oder ihren Vorläufern, oder Syntheseenzymen, trophischen Faktoren, Apolipoproteinen, Dystrophin oder Minidystrophin, Tumorsuppressoren und an der Blutgerinnung beteiligten Faktoren, codiert.

10. Rekombinantes Adenovirus mit Ursprung aus einem nicht-menschlichen Tier, enthaltend mindestens eine insertierte Antisensesequenz, mit therapeutischer Wirkung beim Menschen.

11. Adenovirus nach Anspruch 9 oder 10, **dadurch gekennzeichnet, dass** es auch Promotorsequenzen umfasst, die die Expression des insertierten therapeutischen Gens bzw. der insertierten therapeutischen Gene erlauben.

12. Adenovirus nach den Ansprüchen 9 bis 11, **dadurch gekennzeichnet, dass** es auch Signalsequenzon umfasst, die die Induktion einer Sekretion des Expressionsprodukts des therapeutischen Gens erlauben.

13. Adenovirus nach den Ansprüchen 9 bis 12, **dadurch gekennzeichnet, dass** das Adenovirus aus Hunde-, Rinder-, Maus-, Schaf-, Schweine-, Vogel- und Affen-Adenoviren ausgewählt ist.

14. Adenovirus nach Anspruch 13, **dadurch gekennzeichnet, dass** das Adenovirus ein Hunde-Adenovirus, bevorzugt ausgewählt aus den Stämmen Adenovirus CAV-2, ist.

15. Adenovirus nach Anspruch 14, **dadurch gekennzeichnet, dass** es Regionen eines anderen tierischen oder menschlichen Adenovirus umfasst.

16. Pharmazeutische Zusammensetzung, umfassend ein oder mehrere Adenoviren nach einem der Ansprüche 9 bis 15.

## Claims

1. Use of a recombinant adenovirus of non-human animal origin, having a genome which lacks the sequences needed for replication, and containing a heterologous DNA sequence for the preparation of a pharmaceutical composition intended for the therapeutic and/or surgical treatment of human body.

2. Use according to Claim 1, for the preparation of a pharmaceutical composition intended for the transfer of therapeutic genes in humans.

3. Use according to Claim 1, for the preparation of a vaccine.

4. Use according to Claim 2, **characterized in that** the therapeutic gene is a gene coding for a therapeutic proteinaceous product.

5. Use according to Claim 2, **characterized in that** the therapeutic gene is an antisense gene or sequence.

6. Use according to one of Claims 1 to 5, **characterized in that** the adenovirus is chosen from canine, bovine, murinc, ovine, porcine, avian and simian adenoviruses.

7. Use according to Claim 6, **characterized in that** the adenovirus is a canine adenovirus, preferably chosen from the strains of CAV-2 adenovirus.

8. Use according to claim 7, **characterized in that** the adenovirus genome lacks the E1A and E1B regions.

9. Recombinant adenovirus of non-human animal origin, having a genome which lacks at least the sequences needed for replication, and containing at least one inserted gene coding for a protein product having a therapeutic effect in humans chosen from enzymes, blood derivatives, hormones, lymphokines, growth factors, neurotransmitters or their precursors or synthetic enzymes, trophic factors, apolipoproteins, dystrophin or minidystrophin, tunaour-suppressors and factors involved in coagulation.

10. Recombinant adenovirus of non-human animal origin containing at least one inserted antisense sequence which has a therapeutic effect in humans.

11. Adenovirus according to Claim 9 or 10, **characterized in that** it also comprises promoter sequences permitting expression of the inserted therapeutic gene or genes.

12. Adenovirus according to one of Claims 9 to 11, **characterized in that** it also comprises signal sequences enabling a secretion of the expression product of the therapeutic gene in humans to be induced.

13. Adenovirus according to one of Claims 9 to 12, **characterized in that** the adenovirus is chosen from canine, bovine, murine, ovine, porcine, avian and simian adenoviruses.

14. Adenovirus according to Claim 13, **characterized in that** the adenovirus is a canine adenovirus, preferably chosen from the stains of CAV-2 adenovirus.

15. Adenovirus according to Claim 14, **characterized in that** it comprises region of another human or animal adenovirus.

16. Pharmaceutical composition comprising one or more adenoviruses according to one of the Claims 9 to 15.
